Europäisches Patentamt

(19) European Patent Office

Office européen des brevets

(11) EP 0 658 321 B1

(12) EUROPEAN PATENT SPECIFICATION

(45) Date of publication and mention
of the grant of the patent:
09.06.1999 Bulletin 1999/23

(51) Int. Cl.$^6$: **A41D 13/00**, A61F 9/02

(21) Application number: 94250231.1

(22) Date of filing: 22.09.1994

(54) **Disposable aerosol mask with face shield**

Wegwerfschutzmaske mit Gesichtsschild gegen Aerosolteilchen

Masque de protection jetable contre des particules aérosols pourvu d'un bouclier facial

(84) Designated Contracting States:
AT BE CH DE DK ES FR GB GR IE IT LI LU MC NL
PT SE

(30) Priority: 15.12.1993 US 168090

(43) Date of publication of application:
21.06.1995 Bulletin 1995/25

(73) Proprietor:
TECNOL MEDICAL PRODUCTS, INC.
North Richland Hills, Texas 76180 (US)

(72) Inventor: **Brunson, Kevin K.**
**Argyle, TX 76226 (US)**

(74) Representative:
**UEXKÜLL & STOLBERG**
**Patentanwälte**
**Beselerstrasse 4**
**22607 Hamburg (DE)**

(56) References cited:
EP-A- 0 606 686          WO-A-89/10106
WO-A-91/08829           US-A- 4 944 294
US-A- 5 020 533

## Description

### TECHNICAL FIELD OF THE INVENTION

This invention relates generally to a face mask capable of preventing passage of airborne aerosol particles. More particularly, and not by way of limitation, this invention relates to a disposable face mask having a relatively low pressure drop to permit easy breathing, while preventing aerosol particles from passing therethrough and therearound. A visor or shield may be attached to the mask to prevent liquids from contacting the eyes of a wearer.

### BACKGROUND OF THE INVENTION

Disposable masks have been manufactured for many years. In the medical field, most of these masks have been for use in preventing contamination of a patient by the breath of health care personnel. In recent years with increased concern for infection of health care personnel with airborne pathogens, such as the hepatitis B virus, it has become necessary to prevent not only the contamination of patients due to exhalation from health care personnel, but also to prevent infection of health care personnel due to inhalation of airborne infection particles. It has become even more important in view of the advent of human immunodeficiency virus (HIV) and the recent increase in infectious tuberculosis associated with many HIV patients.

In addition, it has been found that aerosols having airborne liquid and solid particles are generated not only by the exhalation of infected patients, but also by certain procedural manipulations and processes that impart energy to any microbial suspension. Surgical procedures involving use of drills and saws are particularly prolific producers of the aerosols which may contain tuberculosis, HIV or other pathogens from an infected patient. Concern with tuberculosis has been increasing since new strains of the disease show strong resistances to multiple types of drug treatment.

In addition, it has been shown that many of the viral hemorrhagic fevers such as yellow fever, Rift Valley fever and perhaps Rocky Mountain spotted fever, rabies and smallpox can be transmitted through aerosols. A considerable number of studies have been made which are now beginning to identify the transmission of such viruses through "non-accident" situations. Accordingly, it is now believed that many of those "non-accident" situations result from aerosol contamination.

Of the current medical masks on the market, it appears that many are not effective against aerosols. One of the presently available molded-type surgical masks has almost no resistance to particles smaller than two (2) microns and has a low efficiency in blocking particles as large as nine (9) microns. Some masks apparently demonstrate somewhat better qualities, but none appear to be fully satisfactory in preventing the passage of aerosols through the mask or around the periphery of the mask.

One type of mask is illustrated in U.S. Patent No. 2,012,505 entitled *Mask*, issued on August 27, 1935 to S.J. Goldsmith. Another type of disposable face mask is illustrated in U.S. Patent No. 4,319,567 entitled *Disposable Face Mask*, issued on March 16, 1982 to M. Magidson. This mask is molded and has been especially configured in an effort to avoid leakage of fluid flow past the edges of the mask. Obviously, leakage cannot be tolerated when attempting to control aerosols. U.S. Patent 4,606,341 entitled *Noncollapsible Surgical Face Mask*, issued August 19, 1986 to Vance M. Hubbard and Welton K. Brunson shows a conventional rectangular face mask having a trapezoidal pleat. Rectangularly shaped masks, including the mask shown in Patent '341, have less than an optimal fit to prevent the passage of aerosols between the periphery of the masks and a wearer's face. An additional folded type mask is illustrated in U.S. Patent No. 4,688,566 entitled *Filter Mask*, issued on August 25, 1987 to Elvin L. Boyce.

Recent developments in surgical face mask have resulted in improved resistance to liquid penetration from the exterior of such masks. Visors or face shields are often attached to such surgical masks to protect the eyes of a wearer. U.S Patent No. 4,920,960 entitled *Body Fluids Barrier Mask*, issued on May 1, 1990 to Hubbard, *et al.*, is exemplary of improvements in such masks. U.S. Patent No. 5,020,533 entitled *Face Mask with Liquid and Glare Resistant Visor*, issued on June 4, 1991 to Hubbard, *et al.*, is an example of incorporating a visor to protect the face of a wearer from liquids during medical procedures.

### SUMMARY OF THE INVENTION

In accordance with the present invention, an improved aerosol mask is provided to substantially reduce or eliminate the shortcomings previously associated with aerosol-type face masks. For some applications, a face shield may be attached to the improved aerosol mask in accordance with the present invention. For other applications, the improved aerosol mask may include one or more layers of expanded polytetrafluoroethylene.

An aerosol mask and a face shield incorporating the present invention provide an improved seal with the face of a wearer, higher filtration capability with respect to aerosols, a sufficiently low pressure drop through the mask for comfortable breathing, and improved protection from liquid splashes or liquid sprays.

In one aspect, this invention provides a disposable mask according to claim 1.

In another aspect, the present invention provides a disposable mask according to claim 11.

The present invention has significant technical advantages in that a face mask and visor are provided for forming a barrier with the face of a wearer to prevent passage of aerosols between the periphery of the mask and the wearer's face and to block liquid spray and splashes from contacting portions of the wearer's face which are not covered by the mask. The general trapezoidal shape of the face mask cooperates with inner and outer radii on opposite sides of the mask to provide a relatively flat sealing surface with the face of a wearer. The inner radii portions on opposite sides of the mask cooperate to position the visor relative to the mask and the wearer's eyes. The mask also provides substantially increased flow area for the passage of air through the mask during normal breathing by the wearer while at the same time allowing the use of filtration media having higher resistance to the passage of aerosols through the mask. The present invention allows optimizing the filtration capability for resistance to the passage of aerosols while minimizing the restriction to normal breathing caused by wearing the mask. The present invention also allows for a substantially improved fit between the periphery of the mask and the contours of a wearer's face and positioning an optically clear visor attached to the mask with respect to the eyes and face of the wearer.

An additional technical advantage of the present invention includes attaching a visor to the aerosol mask to eliminate the need for separate eye protection. Curved radii on each side of the mask provide a portion of the means for attaching the visor to the aerosol mask and assist the visor to conform with the wearer's face.

A further technical advantage of the present invention includes a darkened strip of material placed on the top edge of the mask to substantially reduce reflections and glare. The darkened strip of material is particularly beneficial if an optically clear wraparound visor has been attached to the upper portion of the mask to protect the wearer's eyes from liquid splash or spray.

Another technical advantage of the present invention includes the use a filter media formed from expanded polytetrafluoroethylene (PTFE) membrane with one or more layers of bicomponent polyethylene or polypropylene disposed on opposite sides of the filter media.

## BRIEF DESCRIPTION OF THE DRAWINGS

For a more complete understanding of the present invention and the advantages thereof, reference is now made to the following description taken in conjunction with the accompanying drawings, in which:

FIGURE 1 is a perspective view of a mask or respirator with an optically clear visor attached in accordance with the present invention and illustrated on the head of a wearer;

FIGURE 2 is the plan view of the mask and visor of FIGURE 1;

FIGURE 3a is a drawing in section and in elevation with portions broken away of the mask and visor of FIGURE 1 taken generally along lines 3a-3a of FIGURE 2;

FIGURE 3b is an enlarged sectional view with portions broken away of an alternative embodiment of the mask of FIGURE 1 showing four layers of material including at least one liquid impervious layer;

FIGURE 3c is an enlarged sectional view with portions broken away of another alternative embodiment of the mask of FIGURE 1 showing three layers of material including at least one layer of expanded PTFE filter media; and

FIGURE 4 is a plan view of an aerosol mask with an optically clear visor attached in accordance with an alternative embodiment of the present invention.

## DETAILED DESCRIPTION OF THE INVENTION

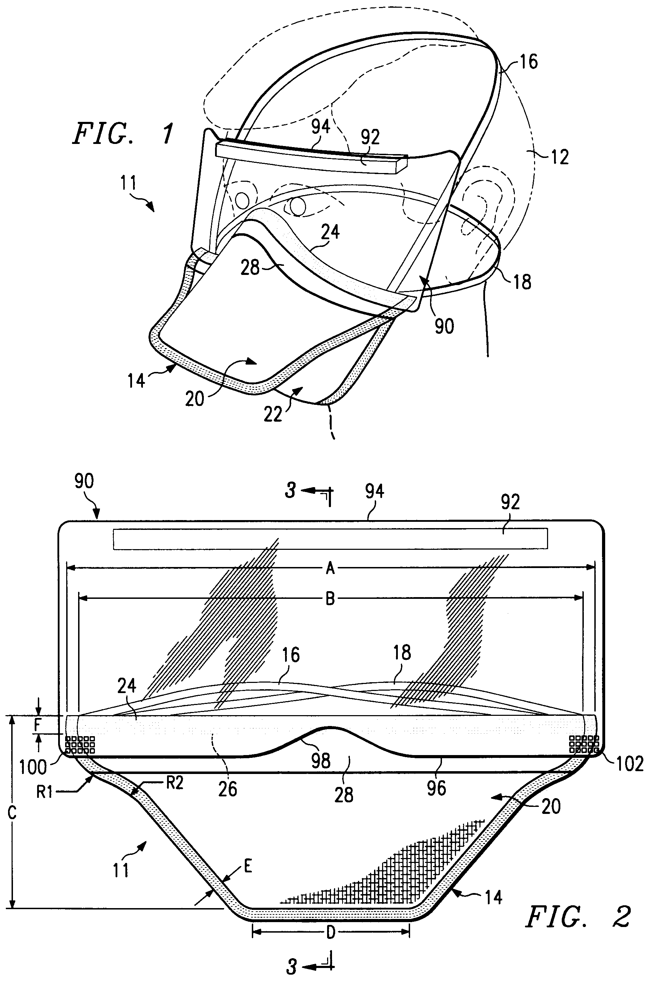

The preferred embodiments of the present invention and its advantages are best understood by referring to FIGURES 1-4 of the drawings, like numerals being used for like and corresponding parts of the various drawings.

Mask 11 with attached visor 90 is illustrated in FIGURE 1 as being positioned on the face of wearer 12 shown in ghost lines. Mask 11 includes filter body 14 which is secured to wearer 12 by means of resilient and elastic straps or securing members 16 and 18. Filter body 14 comprises an upper portion 20 and a lower portion 22 which have a generally trapezoidal configuration. Upper and lower portions 20 and 22 preferably have matching exterior dimensions and shape. Upper and lower portions 20 and 22 may be bonded together by heat and/or ultrasonic sealing along three sides of filter body 14. Bonding in this manner adds important structural integrity to mask 11.

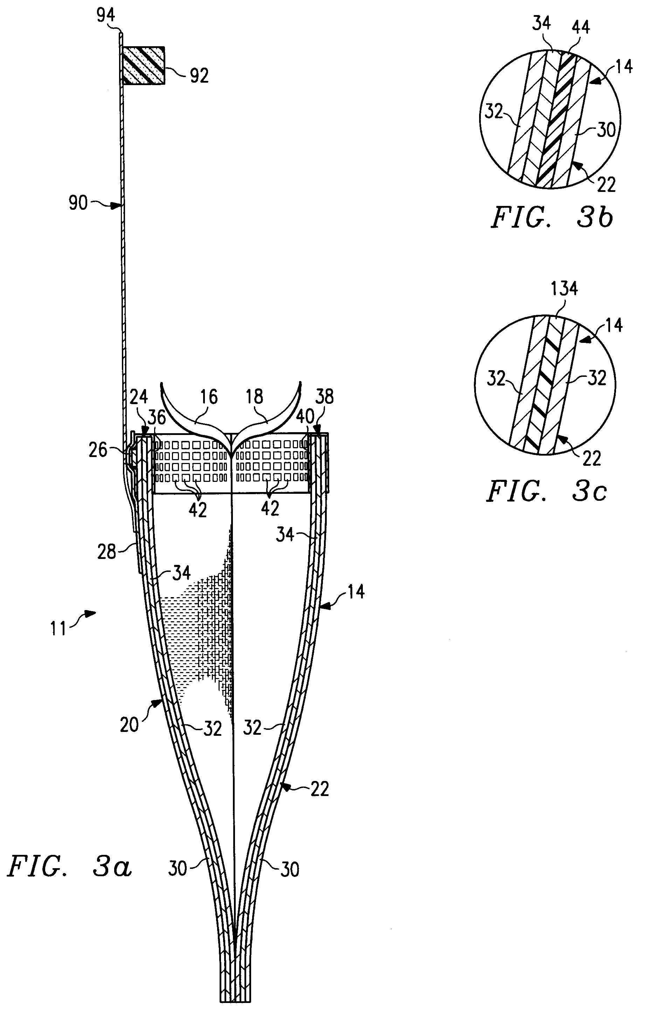

The fourth side of filter body 14 is open and includes a top edge 24 with an elongated malleable member 26 (see FIGURES 2 and 3a). Malleable member 26 is provided so that top edge 24 of mask 11 can be configured to closely fit the contours of the nose and cheeks of wearer 12. Malleable member 26 is preferably constructed from an aluminum strip with a rectangular cross-section, but may also be a moldable or malleable steel or plastic member.

A darkened strip of material 28 may be applied to the exterior of upper portion 20 adjacent to top edge 24. Darkened strip 28 is provided to reduce glare and reflections. The use of darkened strip 28 is particularly beneficial when visor 90 is attached to filter body 14. Darkened strip 28 may comprise any suitable material such as treated color stock. For some applications, darkened strip 28 is preferably black in color and the exterior of upper portion 20 is preferably orange in color.

Top edge 24 of upper portion 20 and bottom edge 38 of lower portion 22 cooperate with each other to define the periphery of mask 11 which contacts the face of wearer 12. The present invention allows optimizing the barrier formed between the periphery of mask 11 and the face of wearer 12 and the filtration capability of mask 11 to resist the passage of particulate matter and aerosols through filtration media 34 while minimizing resistance to normal breathing of wearer 12 resulting from the use of mask 11. The present invention also allows including multiple layers of filter media within filter body 14 including layer 44 of liquid impervious material.

As shown in FIGURES 1 and 3a, mask 11 has the general shape of a cup or cone when placed on the face of wearer 12. The present invention allows mask 11 to provide "off-the-face" benefits of a molded-cone style mask while still being easy to package, store and ship with visor 90 attached to filter body 14. "Off-the-face" style masks provide a larger breathing chamber as compared to soft, pleated masks which contact a substantial portion of the wearer's face. Therefore, "off-the-face" masks permit cooler and easier breathing. The present invention allows optimizing the volume of air contained within filter body 14. If the volume is too large, excessive amounts of exhaled air may be retained within filter body 14 at normal breathing rates. By properly selecting the size of filter body 14, excessive heating of the air within filter body 14 is minimized and dizziness from prolonged periods of rebreathing exhaled air is also minimized.

Face shield or visor 90 preferably comprises a clear, plastic film such as polyester or polyethylene. Visor 90 is generally dimensioned to fit across the portions of the face of wearer 12 which are not covered by mask 11. Visor 90 is specifically positioned on filter body 14 to protect the eyes of wearer 12 from liquid spray and liquid splashes. The plastic material comprising visor 90 may have a thickness of approximately 0.005 inches (0,1 mm) with enough stiffness to prevent collapse and yet having sufficient flexibility to bend and conform generally with the face of wearer 12. As will be explained later in more detail, visor 90 is preferably bonded to filter body 14 at opposite ends of top edge 24.

The approximate optimum dimensions for mask 11 as shown in FIGURE 2 include 10¼" (26 cm) for the major length, dimension A, of the trapezoidal shape of filter body 14. The length of the opening in filter body 14 defined in part by top edge 24 and bottom edge 38, dimension B, is 9 1/4" (23,5 cm) due to approximately 1/2" (1,27 cm) of seal at each junction between upper portion 20 and lower portion 22. The minor length of the parallel portion of trapezoid shaped filter body 14, dimension D, is 3 5/16" (8,4 cm). The opening of filter body 14 defined in part by dimensions A and B is generally parallel with minor length, dimension D. The dimensions A and B may be varied by ± 3/4" (1,9 cm). The width of the trapezoid shape of filter body 14, dimension C, is 3 1/2" (1,27 cm). Dimensions C and D may be varied by ± 1/4" (0,63 cm). The bonded border defining the three closed sides of mask 11, dimension E, is preferably 1/4" (0,63 cm) in width. The portions of top edge 24 and bottom edge 38 which contact the wearer's face, dimension F, are preferably 1/2" (1,27 cm) in width.

Visor 90 preferably has a generally rectangular configuration with rounded corners. The length of visor 90 is preferably selected to be slightly larger than dimension A of filter body 14. The width of visor 90 is selected to be approximately equal to dimension C, the width of filter body 14. Foam strip 92 is preferably secured to the interior surface of visor 90 near upper edge 94. Foam strip 92 is preferably long enough to span the forehead of wearer 12. The width of foam strip 92 is selected to prevent visor 90 from directly contacting the forehead of wearer 12. Foam strip 92 may also be positioned to prevent sweat from dripping into the eyes of wearer 12. Lower edge 96 of visor 90 preferably includes notch 98 which allows visor 90 to conform with the nose and face of wearer 12 without causing creases or wrinkles in visor 90.

The above dimensions may be modified to accommodate wearers having smaller or larger facial features. However, the ratio between the width of the trapezoid shape which defines mask 11, dimension C, as compared to the minor length of the trapezoid shape, dimension D, should preferably remain at approximately 1 to 1. The preferred ratio between the major length of filter body 14, dimension A, and the minor length, dimension D, is approximately 3 to 1. For many applications, the length of visor 90 should remain slightly larger than dimension D and the width of visor 90 approximately equal to dimension C.

An important feature of the present invention is forming radius R1 of approximately 1 1/8" (2,85 cm) and radius R2 of approximately 3" (7,62 cm) in the non-parallel or angled sides of filter body 14. Radii R1 and R2 cooperate with each other to prevent collapse of filter body 14 during normal breathing by wearer 12. These radii, R1 and R2, help mask 11 retain the desired, off-the-face shape during normal breathing. If the sealed border on the three closed sides of filter body 14 is less than 1/4" (0,63 cm), mask 11 may tend to collapse during normal breathing. Therefore, an important feature of the present invention includes combining radii R1 and R2 with a sealed border of approximately 1/4" (0,63 cm) in width to provide the desired cone or cup shaped mask covering the nose and mouth of wearer 12 and to maintain a fluid tight barrier with wearer's face without collapsing during normal breathing.

Radius R2 curves outward from opposite sides of mask 11 and is tangent to radius R1 that curves inward towards the attachment points for headbands 16 and 18 and visor 90. This arrangement is a critical feature of the present invention and serves several important functions. Radii R1 and R2 cooperate with each other to improve the facial seal performance between the periphery of mask 11 and the face of wearer 12.

Radii R1 and R2 allow mask 11 to open with relatively flat surfaces near each end of top edge 24 and bottom edge 38 adjacent to the attachment point for headbands 16 and 18. These flat surfaces adjacent to the attachment points for the headbands 16 and 18 and visor 90 gradually taper away from the opening in filter body 14. The general trapezoidal shape of filter body 14 in cooperation with radii R1 and R2 and the other preferred dimensions and ratios cooperate to minimize collapse of filter body 14 during normal use of mask 11.

Radii R1 and R2 cooperate with top edge portion 24 and bottom edge portion 38 to allow mask 11 to fit securely with and to form a tight facial seal on a greater number of different face sizes. Mask 11 is particularly useful with smaller faces which contact the tapered surfaces adjacent to the attachment points for headbands 16 and 18 on the interior of mask 11 as shown in FIGURE 1. Other types of surgical masks frequently have contact between the periphery of the mask and the skin of the wearer's face at an acute angle with only the narrow, bonded edge of the mask providing a sealing surface.

Radii R1 and R2 assist in maintaining the integrity of mask 11 by providing strength along the three-bonded sides of filter body 14 when mask 11 is fully opened against the face of a wearer. These previously described design features allow for the use of lighter weight or lighter basis material which adds breathability and comfort to mask 11. Without radii R1 and R2 these same lightweight materials would be prone to collapse during inhalation. The 1/4" bonded seal around the three closed sides of filter body 14 contributes to maintaining structural integrity of mask 11 when secured to the face of wearer 12.

Visor 90 is preferably attached to filter body 14 at the opposite ends of top edge 24. Bonded areas 100 and 102 are preferably used to secure visor 90 to filter body 14. Various adhesives, ultrasonic seals and/or heat seals may be used to establish bonded areas 100 and 102. Ultrasonic seals (sometimes referred to as ultrasonic welding) have been found to be particularly advantageous for attaching visor 90 to radii portions R1 of mask 11.

Bonded areas 100 and 102 cooperate with their respective radii portions R1 to allow visor 90 to easily adapt to the contours of the face of wearer 12. Notch 98 in lower edge 96 along with bonded areas 100 and 102 prevent visor 90 from moving up with respect to mask 11 and forming a gap therebetween. Radii portions R1 allow positioning bonded areas 100 and 102 to adjust visor 90 and mask 11 for optimum fit with the face of wearer 12.

Blow-by associated with normal breathing of wearer 12 is substantially eliminated by properly selecting the dimension and location of malleable strip 26 with respect to top edge of 24. Malleable strip 26 is preferably positioned in the center of top edge 24 and has a length in the range of fifty percent to seventy percent of the total length, dimension A, of top edge 24. For one embodiment of the present invention, the performance of mask 11 was enhanced by using malleable strip 26 manufactured from quarter-tempered aluminum. For this embodiment, the length of malleable strip 26 was approximately 54 percent of the length of top edge 24 with a thickness of .021 inches (0,5 mm) and a width of 0.197 inches (0,5 cm).

The present invention allows designing mask 11 with the optimum periphery to fit on the face of wearer 12, the optimum dimension for malleable strip 26 to form an enhanced fluid barrier with the nose and face of wearer 12, and the optimum position for visor 90 with respect to the eyes and uncovered portion of the face of wearer 12. The present invention allows modification to the length of top edge 24 and bottom edge 38 while maintaining approximately the same surface area for normal breathing through filter media 34.

Elastic straps or headbands 16 and 18 are preferably constructed from resilient polyurethane, but may be constructed from elastic rubber, or a covered stretch yarn. The covered stretch yarn may consist of an elastomeric material wrapped with nylon or a polyester. The use of two headbands 16 and 18 substantially improves the fluid barrier between the periphery of mask 11 and the face of wearer 12.

As illustrated in FIGURE 3a, upper and lower portions 20 and 22 each include an outer mask layer 30 that is preferably constructed from a spun-bonded polypropylene. Outer mask layers 30 may also be constructed from a bi-component and/or powder bonded material such as polyethylene or polypropylene, a cellulastic tissue, or a spun-bonded polyester. Outer mask layers 30 typically have a basis weight range of 0.5 ounces per yard (15,5 g/m) to 1.0 ounces per yard (31 g/m). 0.9 ounces per yard (27,9 g/m) is one of the preferred basis weights for outer layers 30.

Inner mask layers 32 are preferably composed of a bicomponent polyethylene and polypropylene. Layers 32 may also be constructed from polyester and/or polyethylene material or cellulastic tissue. Layers 32 typically have a basis weight range of 0.4 ounce per yard (12,4 g/m) to 0.75 ounces per yard. 0.413 ounces per yard (23,2 g/m) is one of the preferred basis weights for layers 32. One or more intermediate layers of filter media may be disposed between outer mask layer 30 and inner mask layer 32. Selection of the number and type of intermediate layers of filter media will depend upon the intended use and function for mask 11.

In FIGURE 3a, filter body 14 is shown with only one intermediate mask layer 34 which comprises the filter media for the associated mask 11. This layer is preferably constructed from a melt-blown polypropylene, but may be constructed from an extruded polycarbonate, a melt-blown polyester, or a melt-blown urethane.

FIGURE 3b shows an alternative embodiment of the present invention in which filter body 14 includes two intermediate layers 34 and 44 of filter media. Layer 44 may be formed from a barrier material that is gas permeable and permits gas (air) to pass through filter body 14 in both directions and is impermeable to liquid passing through mask 11 in at least one direction. Layer 44 is preferably arranged to prevent the passage of liquids from the exterior of filter body 14 through layer 44 to the interior of filter body 14.

A more complete description of the construction and operation of such material can be found in U.S. Patent No. 3,929,135 entitled *Absorptive Structure Having Tapered Capillaries*, issued on December 30, 1975 to Hugh A. Thompson. Such materials are often constructed from a low density polyethylene and include small apertures which prevent liquids from passing therethrough due to the liquid's relatively high surface tension. U.S. Patents '960; '533 and 5,150,703 entitled *Liquid Shield Visor for a Surgical Mask with a Bottom Notch to Reduce Glare*, issued on September 29, 1992 to Hubbard, *et al* provide additional information on materials which may be used for layers 30, 32, 34 and 44 and face masks constructed with such materials. These patents are assigned to Tecnol Medical Products, Inc. Other types of microporous film may be satisfactorily used with the present invention.

The use of barrier materials such as layer 44 is particularly important when mask 11 with visor 90 is worn in an environment where the wearer may be exposed to "body fluids". These fluids such as blood, urine and saliva may contain highly contagious germs and viruses. Contact of AIDS-contaminated body fluids with another person's source of body fluids, such as the eyes, nose and mouth, may transmit the disease. Therefore, it is often preferable to include layer 44 which is resistant to the passage of liquids through filter body 14 to prevent body fluids from contacting the nose and mouth of the wearer.

FIGURE 3c shows another alternative embodiment of the present invention in which filter body 14 includes intermediate layer 134 of filter media disposed between layers 32. For this particular embodiment of the present invention the inner and outer mask layers 32 are formed from the same type of material. However, various types of material may be used with intermediate mask layer 134.

For one embodiment of the present invention intermediate mask layer 134 was formed from an expanded polytetrafluoroethylene (PTFE) membrane. Such materials are manufactured by W. L. Gore & Associates. A more complete description of the construction and operation of such materials can be found in U.S. Patent No. 3,953,566 entitled *Process for Producing Porous Products*, issued on April 27, 1976 to Robert W. Gore, and U.S. Patent No. 4,187,390 entitled *Porous Products and Process Therefor*, issued on February 5, 1980 to Robert W. Gore. For some applications and operating environments the use of filter media 134 substantially enhances the performance of the associated aerosol mask 11.

As demonstrated by the previous comments, the generally trapezoidal shape of filter body 14 including the preferred ratios for the dimensions of filter body 14 and radii R1 and R2 allows a wide variety of materials to be used in the manufacturer of the mask layers which comprise filter body 14. The present invention has significantly increased the types of material which may be satisfactorily used in constructing filter body 14. The present invention also allows more options with respect to selecting the number of layers of material used to manufacture filter body 14.

A mask with the filter media or intermediate layer 34 was selected to test filtration of particle sizes of about 1.0 micron. In tests run using standardized testing procedures for filter materials, filter media 34 had an efficiency in excess of 98 percent. The efficiency is defined by the equation:

$$\% Eff = \frac{Avgc - Avgt}{Avgc} \qquad (1)$$

where:

c is the particle count with no test sample in the path and Avgc is the average particle count of three runs; and
t is the particle count with a test sample in the path and Avgt is the average particle count of three runs.

In the test procedure, 1.0 micron latex particles were aerosolized, dried and passed through the test samples at the rate of one cubic foot per minute. The particles were counted using a laser based particle counter.

Top edge 24 of mask 11 may be faced with an edge binder 36 that extends across the open end of mask 11 and covers malleable strip 26. Similarly, lower portion 22 of mask 11 forms a bottom edge 38 that is encompassed in an edge binder 40. Edge binders 36 and 40 are preferably constructed from a spun-laced polyester material. The binders may also be constructed from a number of thermally bonded bicomponent materials or from polypropylene or polyethylene non-porous plastic films.

Referring to FIGURE 1, mask 11 is illustrated as being located on the face of wearer 12. Upper portion 20 with malleable member 26 located in top edge 24 and lower edge 96 of visor 90 conform very closely to the configuration of the nose and cheeks of wearer 12. It is also important that the fit between bottom edge 38 and the chin of wearer 12 and top edge 24 and the nose and cheeks of the wearer 12 fit very closely since any leaks result in bypass or blow-by of air either entering mask 11 or being discharged from mask 11 as it is used by wearer 12. Also, leakage around top edge 24 may cause fogging of visor 90.

Accordingly, elastomeric headbands or straps 16 and 18 have their ends attached at the junctures between top edge 24 and bottom edge 38 of mask 11 as shown in FIGURE 3a. The arrangement is such that strap 16 can be placed over the top of the head of wearer 12, as illustrated in FIGURE 1, in alignment with bottom edge 38 of mask 11 so that a direct force is exerted along that line urging bottom edge 38 into sealing engagement with the chin of wearer 12. Similarly, strap 18 is positioned around the lower base of the skull and in direct alignment with top edge 24 of mask 11 and thus placing a force thereon which tends to move top edge 24 into tighter sealing engagement with the nose and cheeks of wearer 12. As shown in FIGURE 3a, the ends of straps 16 and 18 are secured at the same location between top edge 24 and bottom edge 38 with no gap between the ends of straps 16 and 18. The position of attaching straps 16 and 18 to filter body 14 in cooperation with radii R1 and R2 results in the optimum pull angle to form a fluid tight barrier between the interior of mask 11 and the face of wearer 12.

It is extremely difficult to construct a mask that will fit the facial configuration of all wearers without constructing the mask specifically for each individual face. Los Alamos National Laboratory has established standards for the testing of face masks that utilize panels of people with different face sizes and configurations. Such facial features represent approximately 95 percent of the working population. Two different panels of people are utilized: one, according to face width and length and the other according to lip length and face length. Twenty-five panelists are utilized in each category.

During a typical test, each panelist dons a mask and a hood is placed over their head with saccharin introduced into the hood. The panelist is then asked to perform a certain routine of exercises. If the panelist tastes the saccharin, the mask fit test is a failure. Mask 11 was subjected to such testing and tested successfully on almost 90 percent of the panelists. Such results are substantially better than any of the current existing masks.

In addition to having a tight peripheral seal, it is essential that mask 11 have good breathability characteristics. That is, mask 11 should require a low differential in pressure to permit air to flow easily through filter body 14 despite the fact that mask 11 will filter 1 micron and smaller particles and have a very tight fit between edges 24 and 38 and the face of wearer 12. A low differential pressure for air flow indicates good breathability through a face mask.

Upper portion 20 and lower portion 22 of mask 11 have a combined surface area through which air can flow of about 250 square centimeters. Thus, body 14 of mask 11 has a surface area of approximately 250 square centimeters which provides enhanced breathability for wearer 12. Tests were run utilizing a flow rate of thirty-two (32) liters per minute across the entire flow area. Approximately thirty masks incorporating the present invention were checked. The masks had a pressure differential ranging from 0.9 to 1.3 mm of water with a mean pressure differential across the mask of about 1.25 mm of water. Such a low differential in pressure across the mask provides excellent breathability characteristics despite the ability of the mask to filter one micron and smaller sized particles with essentially zero edge leakage around the periphery of mask 11.

Elastomeric head bands or straps 16 and 18 may be replaced by surgical tie straps if desired. Also, a veil guard or gap guard may be attached to bottom edge 38 to protect the neck of wearer 12 from undesirable contact with aerosols and body fluids. The use of a veil guard and surgical tie straps are more fully described in parent patent application Serial No. 07/991,154 filed December 16, 1992, entitled *Disposable Aerosol Mask*.

Mask 11 may be assembled using the following process. Each layer 30, 32 and 34 is placed on its appropriate sheet of raw material. Inner mask layer 32 for upper portion 20 is placed in juxtaposition with inner mask layer 32 for lower portion 22. The inner mask layers 32 cooperate with each other to form the inside surface of the respective mask 11. First and second intermediate layers 34 are then placed in juxtaposition with respect to the respective first and second inner mask layers 32. First and second intermediate layers 44 may be included as desired. First outer mask layer 30 for upper portion 20 along with malleable strip 26 is then attached to the respective intermediate layers 34 and/or 44 along with the first inner layer 32 to form top portion 20. Second outer layer 30 is then attached to the respective intermediate layers 34 and/or 44 along with the second inner layer 32 to form bottom portion 22.

Binders 36 and 40 are preferably secured to top edge 24 and bottom edge 38 respectively by a plurality of ultrasonic seals 42. The three sides of upper portion 20 and lower portion 22 are connected with each other by heat sealing or ultrasonic bonding to form filter body 14 having a general trapezoidal shape with an open side defined by top edge 24 and bottom edge 38. Straps 16 and 18 are attached to the corners of top edge 24 and bottom edge 38 at the junction with upper portion 20 and lower portion 22 during ultrasonic bonding of the three sides of filter body 14. Darkened strip 28 is next attached by heat sealing or ultrasonic bonding to top edge 34 and radii portions R1. The remaining edge of darkened strip 28 is not attached to upper portion 20 to avoid limiting the breathability of filter body 14. Finally, visor 90 is attached to upper portion 20 at bonded areas 100 and 102.

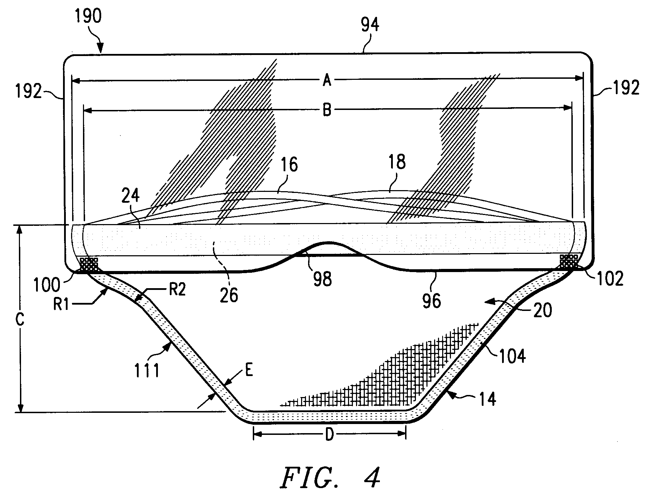

Another alternative embodiment of the present invention is represented by mask 111 with attached visor 190 as shown in FIGURE 4. Mask 111 includes filter body 14 which may be secured to wearer 12 by securing members 16 and 18 as previously described for mask 11. As shown in FIGURE 4, mask 111 does not include darkened strip of material 28. If desired, darkened strip 28 may be included with mask 111 in the same manner as previously described for mask 11.

Face shield or visor 190 is preferably the same as face shield or visor 90 with the exception of foam strip 92. By attaching visor 190 to filter body 14 as shown in FIGURE 4, foam strip 92 is no longer required to prevent visor 190 from directly contacting the forehead of wearer 12. By selecting the proper location for bonds 100 and 102, visor 190 will stand away from the face of a person wearing mask 111.

For one embodiment of the present invention narrow sides 192 of visor 190 are preferably positioned perpendicular to top edge 24 of filter body 14. Lower edge 96 of visor 190 is preferably positioned between 1/2 inch (1,27 cm) and 1 inch (2,54 cm) below the top edge of malleable strip 26. Bonded areas 100 and 102 should extend in from the edge of filter body 14 at least 3/8 of an inch (0,95 cm) but no more than 1/8 of an inch (0,31 cm) past bonded border 104 (dimension E).

Although the present invention has been described in detail, it should be understood that various changes, substitutions and alternations can be made herein without departing from the scope of the invention as defined in the following claims.

## Claims

1. A disposable mask comprising:

   a filter body (14) having an opening sized to cover the nose and mouth of a wearer, said filter body (14) having top (24) and bottom edges (38) edges with said top edge (24) arranged to extend across the nose of said wearer and said bottom edge (38) arranged to extend under the chin of said wearer;

   said top edge (24) having ends opposite from each other and said bottom edge (38) having ends opposite from each other;

   first securing means (18) attached to said filter body (14) adjacent to each end of said top edge (24) and arranged to extend generally about the back of the head of said wearer for urging said top (24) edge into tight engagement with said wearer to prevent fluid flow between said top edge (24) and said wearer;

   second securing means (16) attached to said body adjacent to each end of said bottom edge (38) and arranged to extend generally over the top of the head of said wearer for urging said bottom edge (38) into tight engagement with said wearer to prevent fluid flow between said bottom edge (38) and said wearer;
   characterized in that:

   said first securing means (18) extends in an approximate linear continuation of said top edge (24);

   said second securing means (16) extends in an approximate linear continuation of said bottom edge (38);

   one end of said top edge (24) is bonded with one end of said bottom edge (38) and the other end of said top edge (24) is bonded with the other end of said bottom edge (38) to define in part said opening for said filter body (14); and that

   a visor (90) dimensioned to cover the eyes of said wearer is attached to said filter body (14) proximate the bonded ends of said top edge (24) and bottom edge (38).

2. The mask of Claim 1 wherein said filter body (14) further comprises:

   an upper portion of generally trapezoidal configuration having a longer side forming said top edge (24);

   a lower portion of generally trapezoidal configuration having a longer side forming said bottom edge (38); and

   said upper and lower portions being joined along all remaining sides of said filter body (14).

3. The mask of Claim 2 wherein said filter body further comprises:

   first radii portions (R1) formed on opposite sides of said filter body (14) adjacent to said top edge (24) and said bottom edge (38); and

   said visor (90) bonded with said first radii (R1) on opposite sides of said filter body (14).

4. The mask of Claim 1 wherein said filter body (14) further comprises:

   a plurality of bonds (100, 102) between said visor (90) and said filter body (14); and
   said bonds (100, 102) positioned on said filter body (14) to provide optimum fit between said mask, said visor (90) and said wearer's face.

5. The mask of Claim 1 further comprising an elongated malleable member (26) located in said top edge (24) for conforming said top edge (24) to the contours of said wearer's nose and cheeks.

6. The mask of Claim 5, further comprising said malleable member (26) located in the center of said top edge (24) and having a length corresponding to more than 50% and less than 70% of the length of said top edge.

7. The mask of Claim 1 further comprising a strip of darkened material (28) on the exterior of said filter body (14) adjacent to said visor (90).

8. The mask of Claim 1 wherein said filter body further comprises an intermediate layer of material (34) which is gas permeable in both directions through said filter body (14) and liquid impermeable in the direction from outside said filter body (14) to inside said filter body.

9. The mask of Claim 1 wherein said filter body (14) further comprises an intermediate layer (34, 134) of filter media formed from expanded polytetrafluoroethylene (PTFE).

10. The mask of Claim 9 wherein said filter body (14) further comprises a first layer (30) of bicomponent material disposed on one side of said intermediate layer (34, 134) of expanded polytetrafluoroethylene and a second layer (32) of the same bicomponent material disposed on the opposite side of said layer of expanded polytetrafluoroethylene.

11. A disposable mask comprising:

a filter body (14) having an opening sized to cover the nose and mouth of a wearer, said filter body having top (24) and bottom (38) edges with said top edge arranged (24) to extend across the nose and cheeks of said wearer and said bottom edge (38) arranged to extend under the chin of said wearer;
characterized in that:

said top edge (24) having ends opposite from each other and said bottom edge (38) having ends opposite from each other, said ends of said top edge (24) being joined with said ends of said bottom edge (38) to define in part said opening in said filter body (14);

said filter body (14) comprising an upper portion of trapezoidal configuration having a longer side forming said top edge (24) and a lower portion of trapezoidal configuration having a longer side forming said bottom edge (38);

a plurality of radii (R1, R2) formed on opposite sides of said filter body (14) extending from said top edge (24) and said bottom edge (38); and

a visor (90) dimensioned to cover the eyes of said wearer and attached to said radii (R1, R2) of said filter body (14) proximate said opposite ends of said top edge (24).

12. The mask of Claim 11 wherein said filter body (14) further comprises:

first securing means (18) attached to said filter body (14) adjacent to each end of said top edge (24) and positioned to extend generally about the back of said head of said wearer in an approximate linear continuation of said top edge (24), said first securing means (18) for urging said top edge (24) into tight engagement with said wearer for preventing fluid flow between said top edge (24) and said wearer; and

second securing means (16) attached to said filter body (14) adjacent to each end of said top edge (24) and positioned to extend generally over the top of said head of said wearer in an approximate linear continuation of said bottom edge (38), said second securing means (16) for urging said bottom edge (38) into tight engagement with said wearer for preventing fluid flow between said bottom edge (38) and said wearer.

13. The mask of Claim 11 further comprising an elongated malleable member (26) located in said top edge (24) for conforming said top edge (24) to the contours of said wearer's nose and cheeks.

14. The mask of Claim 11 wherein said filter body (14) further comprises:

first portions with a first radius (R1) that curves inward towards said top edge (24);

second portions with a second radius (R2) that curves outward from said first portion of said filter body (14); and

a plurality of bonds (100, 102) between said visor (90) and said first portions of said filter body (14).

15. The mask of Claim 14 wherein said filter body (14) further comprises said second radius (R2) tangent to said first radius (R1).

16. The mask of Claim 11 wherein said filter body (14) comprises a layer of filter media (34, 134) for restricting the flow of liquids through said filter body (14).

17. The mask of Claim 11 wherein said filter body (14) further comprises an intermediate layer (34, 134) of filter media formed from expanded polytetrafluoroethylene (PTFE).

18. The mask of Claim 17 wherein said filter body further comprises a first layer (30) of bicomponent material disposed on one side of said intermediate layer (34, 134) of expanded polytetrafluoroethylene and a second layer (32) of the same bicomponent material disposed on the opposite side of said layer of expanded polytetrafluoroethylene.

**Patentansprüche**

1. Wegwerfbare Schutzmaske, mit:

einem Filterkörper (14) mit einer Öffnung, die bemessen ist, um die Nase und den Mund eines Trägers zu überdecken, wobei der Filterkörper (14) eine obere (24) und eine untere (38) Kante hat, wobei die obere Kante (24) dazu ausgestaltet ist, sich über die Nase des Trägers zu erstrecken, und die untere Kante (38) dazu ausgestaltet ist, sich unter das Kinn des Trägers zu erstrecken;
wobei die obere Kante (24) Enden hat, die voneinander gegenüberliegend sind, und die untere Kante (38) Enden hat, die voneinander gegenüberliegend sind;
einer ersten Befestigungseinrichtung (18), die an dem Filterkörper (14) benachbart zu jedem Ende der oberen Kante (24) angebracht und dazu ausgestaltet ist, sich allgemein um den hinteren Bereich des Kopfes des Trägers zu erstrecken, um die obere Kante (24) in festsitzende Anlage mit dem Träger zu drücken, um Fluidströmung zwischen der oberen Kante (24) und dem Träger zu verhindern;
einer zweiten Befestigungseinrichtung (16), die an dem Körper benachbart zu jedem Ende der unteren Kante (38) angebracht und dazu ausgestaltet ist, sich allgemein über den oberen Bereich des Kopfes des Trägers zu erstrecken, um die untere Kante (38) in festsitzende Anlage mit dem Träger zu drücken, um Fluidströmung zwischen der unteren Kante (38) und dem Träger zu verhindern;
dadurch gekennzeichnet, daß:
sich die erste Befestigungseinrichtung (18) in einer ungefähr linearen Fortsetzung zu der oberen Kante (24) erstreckt;
sich die zweite Befestigungseinrichtung (16) in einer ungefähr linearen Fortsetzung zu der unteren Kante (38) erstreckt;
ein Ende der oberen Kante (24) mit einem Ende der unteren Kante (38) verbunden und das andere Ende der oberen Kante (24) mit dem anderen Ende der unteren Kante (38) verbunden ist, um teilweise die Öffnung für den Filterkörper (14) zu bilden; und daß
eine Blende (90), die bemessen ist, um die Augen des Trägers zu überdecken, an dem Filterkörper (14) nahe der verbundenen Enden der oberen Kante (24) und der unteren Kante (38) angebracht ist.

2. Schutzmaske nach Anspruch 1, bei der der Filterkörper (14) außerdem aufweist:

einen oberen Bereich mit allgemein trapezförmiger Ausgestaltung, der eine längere Seite hat, durch die die obere Kante (24) gebildet ist;
einen unteren Bereich mit allgemein trapezförmiger Ausgestaltung, der eine längere Seite hat, durch die die untere Kante (38) gebildet ist; und
der obere und untere Bereich entlang aller übrigen Seiten des Filterkörpers (14) miteinander verbunden sind.

3. Schutzmaske nach Anspruch 2, bei der der Filterkörper außerdem aufweist:

erste Radien-Bereiche (R1), die an gegenüberliegenden Seiten des Filterkörpers (14) benachbart zu der oberen Kante (24) und der unteren Kante (38) gebildet sind; und
die Blende (90) mit dem ersten Radius (R1) an gegenüberliegenden Seiten des Filterkörpers (14) verbunden ist.

4. Schutzmaske nach Anspruch 1, bei der der Filterkörper (14) außerdem aufweist:

eine Anzahl von Verbindungen (100, 102) zwischen der Blende (90) und dem Filterkörper (14); und
die Verbindungen (100, 102) an dem Filterkörper (14) angeordnet sind, um einen optimalen Sitz zwischen der Schutzmaske, der Blende (90) und dem Gesicht des Trägers zu bewirken.

5. Schutzmaske nach Anspruch 1, die außerdem ein längliches, formbares Bauteil (26) aufweist, das in der oberen Kante (24) angeordnet ist, um die obere Kante (24) an die Konturen der Nase und der Wangen des Trägers anzupassen.

6. Schutzmaske nach Anspruch 5, die außerdem das formbare Bauteil (26) aufweist, das in der Mitte der oberen Kante (24) angeordnet ist und eine Länge hat, die mehr als 50% und weniger als 70% der Länge der oberen Kante entspricht.

7. Schutzmaske nach Anspruch 1, die außerdem einen Streifen aus abgedunkeltem Material (28) an der Außenseite des Filterkörpers (14) nahe der Blende (90) aufweist.

8. Schutzmaske nach Anspruch 1, bei der der Filterkörper außerdem eine Zwischenschicht aus Material (34) aufweist, das in beiden Richtungen durch den Filterkörper (14) gasdurchlässig ist und in der Richtung von der Außenseite des Filterkörpers (14) zu der Innenseite des Filterkörpers flüssigkeitsundurchlässig ist.

9. Schutzmaske nach Anspruch 1, bei der der Filterkörper (14) außerdem eine Zwischenschicht (34, 134) aus Filtermedium aufweist, das aus gestrecktem Polytetrafluorethylen (PTFE) gebildet ist.

10. Schutzmaske nach Anspruch 9, bei der der Filterkörper (14) außerdem eine erste Schicht (30) aus Bikomponentenmaterial, die an einer Seite der Zwischenschicht (34, 134) aus gestrecktem Polytetrafluorethylen vorgesehen ist, und eine zweite Schicht (32) aus dem gleichen Bikomponentenmaterial aufweist, die an der gegenüberliegenden Seite der Schicht aus gestrecktem Polytetrafluorethylen vorgesehen ist.

11. Wegwerfbare Schutzmaske, mit:

einem Filterkörper (14) mit einer Öffnung, die bemessen ist, um die Nase und den Mund eines Trägers zu überdecken, wobei der Filterkörper eine obere (24) und untere (38) Kante hat, wobei die obere Kante (24) dazu ausgestaltet ist, sich über die Nase und die Wangen des Trägers zu erstrecken, und die untere Kante (38) dazu ausgestaltet ist, sich unter das Kinn des Trägers zu erstrecken; dadurch gekennzeichnet, daß: die obere Kante (24) Enden hat, die voneinander gegenüberliegend sind, und die untere Kante (38) Enden hat, die voneinander gegenüberliegend sind, wobei die Enden der oberen Kante (24) mit den Enden der unteren Kante (38) verbunden sind, um teilweise die Öffnung in dem Filterkörper (14) zu bilden; der Filterkörper (14) einen oberen Bereich mit trapezförmiger Ausgestaltung, der eine längere Seite hat, durch die die obere Kante (24) gebildet ist, und einen unteren Bereich mit trapezförmiger Ausgestaltung aufweist, der eine längere Seite hat, durch die die untere Kante (38) gebildet ist; eine Anzahl von Radien (R1, R2) an gegenüberliegenden Seiten des Filterkörpers (14) gebildet sind, die sich von der oberen Kante (24) und der unteren Kante (38) erstrecken; und

eine Blende (90) bemessen ist, um die Augen des Trägers zu überdecken, und an den Radien (R1, R2) des Filterkörpers (14) nahe den gegenüberliegenden Enden der oberen Kante (24) angebracht ist.

12. Schutzmaske nach Anspruch 11, bei der der Filterkörper (14) außerdem aufweist:

eine erste Befestigungseinrichtung (18), die an dem Filterkörper (14) benachbart zu jedem Ende der oberen Kante (24) angebracht und angeordnet ist, um sich allgemein um den hinteren Bereich des Kopfes des Trägers in einer ungefähr linearen Fortsetzung zu der oberen Kante (24) zu erstrecken, wobei die erste Befestigungseinrichtung (18) die obere Kante (24) in festsitzende Anlage mit dem Träger drückt, um Fluidströmung zwischen der oberen Kante (24) und dem Träger zu verhindern; und eine zweite Befestigungseinrichtung (16), die an dem Filterkörper (14) benachbart zu jedem Ende der oberen Kante (24) angebracht und angeordnet ist, um sich allgemein über den oberen Bereich des Kopfes des Trägers in einer ungefähr linearen Fortsetzung zur unteren Kante (38) zu erstrecken, wobei die zweite Befestigungseinrichtung (16) die untere Kante (38) in festsitzende Anlage mit dem Träger drückt, um Fluidströmung zwischen der unteren Kante (38) und dem Träger zu verhindern.

13. Schutzmaske nach Anspruch 11, die außerdem ein längliches, formbares Bauteil (26) aufweist, das in der oberen Kante (24) angeordnet ist, um die obere Kante (24) an die Konturen der Nase und der Wangen des Trägers anzupassen.

14. Schutzmaske nach Anspruch 11, bei der der Filterkörper (14) außerdem aufweist:

erste Bereiche mit einem ersten Radius (R1), der nach innen in Richtung auf die obere Kante (24) gekrümmt ist; zweite Bereiche mit einem zweiten Radius (R2), der von dem ersten Bereich des Filterkörpers (14) nach außen gekrümmt ist; und eine Anzahl von Verbindungen (100, 102) zwischen der Blende (90) und den ersten Bereichen des Filterkörpers (14).

15. Schutzmaske nach Anspruch 14, bei der der Filterkörper (14) außerdem den zweiten Radius (R2) aufweist, der tangential zu dem ersten Radius (R1) ist.

16. Schutzmaske nach Anspruch 11, bei der der Filterkörper (14) eine Schicht aus Filtermedium (34, 134) aufweist, um die Strömung von Flüssigkeiten durch den Filterkörper (14) zu begrenzen.

17. Schutzmaske nach Anspruch 11, bei der der Filterkörper (14) außerdem eine Zwischenschicht (34, 134) aus Filtermedium aufweist, das aus gestrecktem Polytetrafluorethylen (PTFE) gebildet ist.

18. Schutzmaske nach Anspruch 17, bei der der Filterkörper außerdem eine erste Schicht (30) aus Bikomponentenmaterial, die an einer Seite der Zwischenschicht (34, 134) aus gestrecktem Polytetrafluorethylen vorgesehen ist, und eine zweite Schicht (32) aus dem gleichen Bikomponentenmaterial aufweist, die an der gegenüberliegenden Seite der Schicht aus gestrecktem Polytetrafluorethylen vorgesehen ist.

## Revendications

1. Masque jetable comprenant :

   un filtre (14) ayant une ouverture dimensionnée pour couvrir le nez et la bouche d'un porteur, ledit filtre (14) ayant des arêtes supérieure (24) et des arêtes inférieures (38) avec ladite arête supérieure (24), ladite arête supérieure arrangée pour s'étendre en travers du nez dudit porteur et ladite arête inférieure (38) arrangée pour s'étendre sous le menton dudit porteur ;
   ladite arête supérieure (24) ayant des extrémités opposées l'une à l'autre et ladite arête inférieure (38) ayant des extrémités opposées l'une à l'autre ;
   des premiers moyens de fixation (18) attachés audit filtre (14) adjacents à chaque extrémité de ladite arête supérieure (24) et arrangés pour s'étendre de manière générale sur le derrière de la tête dudit porteur pour pousser ladite arête supérieure (24) en prise serrée avec ledit porteur pour empêcher que du liquide ne s'écoule entre ladite arête supérieure (24) et ledit porteur ;
   des seconds moyens de fixation (16) attachés audit filtre adjacents à chaque extrémité de ladite arête inférieure (38) et arrangés pour s'étendre de manière générale sur le dessus de la tête dudit porteur pour pousser ladite arête inférieure (38) en prise serrée avec ledit porteur pour empêcher que du liquide ne s'écoule entre ladite arête inférieure (38) et ledit porteur ;
   caractérisé en ce que
   lesdits premiers moyens de fixation (18) s'étendent en continuation linéaire approximative de ladite arête supérieure (24) ;
   lesdits seconds moyens de fixation (16) s'étendent en continuation linéaire approximative de ladite arête inférieure (38) ;
   une extrémité de ladite arête supérieure (24) est collée à une extrémité de ladite arête inférieure (38) et l'autre extrémité de ladite arête supérieure (24) est collée à l'autre extrémité de ladite arête inférieure (38) pour définir en partie ladite ouverture pour ledit filtre (14) et
   qu'une visière (90) dimensionnée pour couvrir les yeux dudit porteur est attachée audit filtre (14) près des extrémités collées de ladite arête supérieure (24) et de ladite arête inférieure (38).

2. Masque selon la revendication 1 dans lequel ledit filtre (14) comprend de plus :

   une portion supérieure de configuration généralement trapézoïdale ayant un côté plus long formant ladite arête supérieure (24),
   une portion inférieure de configuration généralement trapézoïdale ayant un côté plus long formant ladite arête inférieure (38) et
   lesdites portions supérieure et inférieure étant réunies le long de tous les côtés restants dudit filtre (14).

3. Masque selon la revendication 2 dans lequel ledit filtre comprend de plus :

   des premières portions de rayon (R1) formées sur les côtés opposés dudit filtre (14) adjacentes à ladite arête supérieure (24) et à ladite arête inférieure (38) et
   ladite visière (90) collée aux premières portions de rayon (R1) sur les côtés opposés dudit filtre (14).

4. Masque selon la revendication 1 dans lequel ledit filtre (14) comprend de plus :

   une pluralité d'attaches (100, 102) entre ladite visière (90) et ledit filtre (14) et
   lesdites attaches (100, 102) positionnées sur ledit filtre (14) pour procurer un ajustement optimal entre ledit masque, ladite visière (90) et le visage dudit porteur.

5. Masque selon la revendication 1 comprennant de plus un organe malléable allongé (26) situé dans ladite arête supérieure (24) pour adapter ladite arête supérieure (24) aux contours du nez et des joues dudit porteur.

6. Masque selon la revendication 5 comprenant de plus ledit organe malléable (26) situé au centre de ladite arête supérieure (24) et ayant une longueur correspondant à plus de 50% et à moins de 70% de la longueur de ladite arête supérieure.

7. Masque selon la revendication 1 comprenant de plus une bande de matière rendue sombre (28) sur l'extérieur dudit filtre (14) adjacente à ladite visière (90).

8. Masque selon la revendication 1 dans lequel ledit filtre comprend de plus une couche intermédiaire de matière (34) qui est perméable au gaz dans les deux sens à travers ledit filtre (14) et qui est imperméable au liquide dans le sens de l'extérieur dudit filtre (14) vers l'intérieur dudit filtre.

9. Masque selon la revendication 1 dans lequel ledit filtre (14) comprend de plus une couche intermédiaire (34, 134) de milieu filtrant formée par du polytétrafluoroéthylène (PTFE) expansé.

10. Masque selon la revendication 9 dans lequel ledit filtre (14) comprend de plus une première couche de matière biconstituante placée sur un côté de ladite couche intermédiaire (34, 134) de polytétrafluoroéthylène expansé et une seconde couche (32) de la même matière biconstituante placée sur le côté opposé de ladite couche de polytétrafluoroéthylène expansé.

11. Masque jetable comprenant :

un filtre (14) ayant une ouverture dimensionnée pour couvrir le nez et la bouche d'un porteur, ledit filtre (14) ayant des arêtes supérieures (24) et des arêtes inférieures (38) avec ladite arête supérieure (24) arrangées pour s'étendre en travers du nez et des joues dudit porteur et ladite arête inférieure (38) étant arrangée pour s'étendre sous le menton dudit porteur ;
caractérisé en ce que
ladite arête supérieure (24) ayant des extrémités opposées l'une à l'autre et ladite arête inférieure (38) ayant des extrémités opposées l'une à l'autre, lesdites extrémités de ladite arête supérieure (24) étant assemblées aux dites extrémités de ladite arête inférieure (38) pour définir en partie ladite ouverture dans ledit filtre (14) ;
ledit filtre (14) comprenant une portion supérieure de configuration généralement trapézoïdale ayant un côté plus long formant ladite arête supérieure (24) et une portion inférieure de configuration généralement trapézoïdale ayant un côté plus long formant ladite arête inférieure (38) ;
une pluralité de rayons (R1, R2) formés sur les côtés opposés dudit filtre (14) s'étendant à partir de ladite arête supérieure (24) et de ladite arête inférieure (38) et
une visière (90) dimensionnée pour couvrir les yeux dudit porteur et attachée aux rayons (R1, R2) dudit filtre (14) près des extrémités opposées de ladite arête supérieure (24).

12. Masque selon la revendication 11 dans lequel ledit filtre (14) comprend de plus :

des premiers moyens de fixation (18) attachés audit filtre (14) adjacents à chaque extrémité de ladite arête supérieure (24) et positionnés pour s'étendre de manière générale sur le derrière de ladite tête dudit porteur en continuation linéaire approximative de ladite arête supérieure (24), lesdits premiers moyens de fixation (18) étant destinés à pousser ladite arête supérieure (24) en prise serrée avec ledit porteur pour empêcher que du liquide ne s'écoule entre ladite arête supérieure (24) et ledit porteur ;
des seconds moyens de fixation (16) attachés audit filtre (14) adjacents à chaque extrémité de ladite arête supérieure (24) et positionnés pour s'étendre de manière générale sur le dessus de ladite tête dudit porteur en continuation linéaire approximative de ladite arête inférieure (38), lesdits seconds moyens de fixation (16) étant destinés à pousser ladite arête inférieure (38) en prise serrée avec ledit porteur pour empêcher que du liquide ne s'écoule entre ladite arête inférieure (38) et ledit porteur.

13. Masque selon la revendication 11 comprenant de plus un organe malléable allongé (26) situé dans ladite arête supérieure (24) pour adapter ladite arête supérieure (24) aux contours du nez et des joues dudit porteur.

14. Masque selon la revendication 11 dans lequel ledit filtre (14) comprend de plus :

des premières portions avec un premier rayon (R1) qui est courbe vers l'intérieur vers ladite arête supérieure (24) ;
des secondes portions avec un second rayon (R2) qui est courbe vers l'extérieur à partir de ladite première portion dudit filtre (14) et
une pluralité d'attaches (100, 102) entre ladite visière (90) et lesdites premières portions dudit filtre (14).

15. Masque selon la revendication 14 dans lequel ledit filtre (14) comprend de plus ledit second rayon (R2) tangent audit premier rayon (R1).

16. Masque selon la revendication 11 dans lequel ledit filtre (14) comprend une couche de milieu filtrant (34, 134) pour restreindre le flux de liquides à travers ledit filtre (14).

17. Masque selon la revendication 11 dans lequel ledit filtre (14) comprend de plus une couche intermédiaire (34, 134) de milieu filtrant formé par du polytétrafluoroéthylène (PTFE) expansé.

18. Masque selon la revendication 17 dans lequel ledit filtre comprend de plus une première couche de matière biconstituante placée sur un côté de ladite couche intermédiaire (34, 134) de polytétrafluoroéthylène expansé et une seconde couche (32) de la même matière biconstituante placée sur le côté opposé de ladite couche de polytétrafluoroéthylène expansé.

FIG. 1

FIG. 2

FIG. 3a

FIG. 3b

FIG. 3c

*FIG. 4*